# EUROPÄISCHE PATENTANMELDUNG

(11) **EP 0 582 321 A1**
(43) Veröffentlichungstag der Anmeldung: **09.02.1994**
(21) Anmeldenummer: 93116081.6
(22) Anmeldetag: 15.01.1993
(51) Int. Cl.: A61K 31/22

(54) **Kombinationspräparat auf der Basis von Dipivalylepinephrin (DPE) und Betablocker zur Augeninnendrucksenkung**

(30) Priorität: 17.01.1992 DE 4201079
(62) Teilanmeldung aus: 93100582.1
(71) Anmelder: Gramer, Eugen, Prof.Dr.med.Dr.jur., D-97080 Würzburg (DE)
(72) Erfinder: Gramer, Eugen, Prof.Dr.med.Dr.jur., D-97080 Würzburg (DE)
(74) Vertreter: von Füner, Alexander, Dr.

(57) **Zusammenfassung**

Kombinationspräparat zur Senkung des Augeninnendrucks, bestehend aus einer Dipivalylepinephrin-Betablocker-Kombination.

Das Kombinationspräparat kann sowohl als Lösung als auch in Suspensionstechnologie hergestellt werden und muß maximal 2 x täglich appliziert werden.

## Beschreibung

Die Erfindung bezieht sich auf ein Kombinationspräparat auf der Basis von DPE und Betablocker zur lokalen Anwendung am Auge zur Senkung des krankhaft erhöhten Augeninnendrucks bei Glaukom. Dipivalylepinephrin (DPE) ist Bestandteil der deutschen Arzneimittel D-Epifrin 0,1 %® und Glaucothil O,1%® und Bestandteil der Kombinationspräparate Thiloadren® und Thilodigon 0,5 %® (Patentschrift DE 30 33 898 C2). Das erfindungsgemäße Kombinationspräparat enthält DPE und als zweite Wirkstoffkomponente einen Betablocker (Beta 1-Beta 2-Blocker oder selektiven Beta-1-Blocker.

Das neue Kombinationspräparat (fixe Kombination) hat eine ganze Reihe von Vorteilen (siehe unten) gegenüber der freien Kombination der Substanzen und wird anfangs 2 x täglich, nach ca. 10 Tagen nur noch 1 x täglich (morgens) appliziert.

### Stand der Technik:

Bisher sind folgende Kombinationspräparate bekannt:
Direktes Parasympathomimetikum/Direktes Sympatholytikum:
Pilocarpin 2 % und Metipranolol 0,1 % (Normoglaucon®)
Pilocarpin 2 % und Timolol 0,5 % (Timpilo®)
Direktes Parasympathomimetikum/Direktes Sympathomimetikum:
Pilocarpin 1 % und Dipivalylepinephrin 0,1 % (Thiloadren®)
Pilocarpin 2 % und Epinephrin 2 % (Piladren®)
Indirektes Sympatholytikum/Direktes Sympathomimetikum:
Guanethidin 0,5 % und Dipivalylepinephrin 0,1 % (Thilodigon® 0,5 %)
Guanethidin 1 % und Adrenalin 0,2 % (Suprexon®)
Guanethidin 3 % und Adrenalin 0,5 % (Suprexon®) Direktes Parasympathomimetikum/reversibler Cholinesterase
hemmer:
Pilocarpin 2 % und Neostigmin 1 % (Syncarpin®)
Pilocarpin 2 % und Physostigmin 0,25 % (Isopto-Pilomin®)
Pilocarpin 2 % und Physostigmin 0,5 % (Pilo-Eserin®)
Der Erfindung liegt die Aufgabe zugrunde, ein Kombinationspräparat zur Verfügung zu stellen, mit dem im Vergleich zur Gabe der Einzelpräparate eine verstärkte Senkung des Augeninnendrucks erreicht werden kann.

Diese Aufgabe wird wie aus den nachstehenden Ansprüchen ersichtlich gelöst.

Gegenstand der Erfindung ist also ein Präparat zur Senkung des Augeninnendrucks auf der Basis von Dipivalylepinephrin, das als zweiten Bestandteil Betablocker enthält.

Es ist bevorzugt, daß das DPE in einer Konzentration von 0,1 bis 0,09 % vorliegt.

Weiterhin ist es bevorzugt, als Betablocker Beta-1-Beta-2-Blocker oder einen selektiven Beta-1-Blocker in einer Konzentration von 0,5 bis 0,25 % einzusetzen.

Entsprechend der Erfindung können die Kombinationspräparate in Form von Augentropfen (Lösung oder Suspension), Augensalbe, Augen-Gel, Augeninsert oder auf einem sonstigen Medikamententräger zur lokalen Applikation am Auge eingesetzt werden.

Dabei ist bei dem erfindungsgemäßen Präparat die Handhabung vereinfacht (nur noch 1 Fläschchen) und so die Gefahr fehlerhafter Anwendung geringer.

Das erfindungsgemäße Kombinationspräparat besteht aus der Kombination der folgenden Wirkstoffgruppen:

### Kombinationspräparat:

Direktes Sympathomimetikum/Direktes Sympatholytikum Dipivalylepinephrin 0,1 % oder weniger (Untergrenze 0,09 %) und Betablocker, z.B. Betaxolol-S 0,28 % oder nicht selektiver Beta-1-Beta-2-Blocker (Suspension)
oder:
Dipivalylepinephrin 0,1 % oder weniger (Untergrenze 0,09 %) und Betablocker, z.B. Betaxolol 0,5 % (Lösung) oder nicht selektiver Beta-1-Beta-2-Blocker (Lösung).

### Beispiele:

Dipivalylepinephrin 0,09 %
mit Betaxolol-Suspension 0,28 %
bzw. Betaxolol-Lösung 0,5 %.

Dipivalylepinephrin 0,1 % (Untergrenze: 0,09 %) mit wahlweise folgenden Betablockern als Lösung:
- Befunolol 0,5 % bzw. 0,25 % als Untergrenze
- Levobunolol 0,5 % bzw. 0,25 % als Untergrenze
- Pindolol 0,5 % bzw. 0,25 % als Untergrenze
- Timolol 0,5 bzw. 0,25 % als Untergrenze
- Carteolol 2 % bzw. 1 % als Untergrenze
- Metipranolol 0,3 % bzw. 0,1 % als Lösung
Bei Anwendung der Suspensionstechnologie kann bei gleicher Drucksenkung die Konzentration in den genannten Grenzen reduziert, maximal halbiert werden.

Das Kombinationspräparat aus DPE und Betablocker kann als Lösung oder in Suspensionstechnologie hergestellt werden. Die Suspensionstechnologie in einem Kombinationspräparat führt für beide Substanzen (Komponenten) zu einer besseren Penetration und langsameren Wirkstoffabgabe (Bolus-Charakter der Medikamente entfällt) und damit wiederum zu einer höheren Bioverfügbarkeit mit höherer Wahrscheinlichkeit der konstanten Augeninnendrucksenkung bei 2 x täglicher Applikation bzw. nach 10 Tagen nur noch 1 x täglich morgens ohne Einschränkung der Wirkungsdauer und Wirkungsstärke im Vergleich zur freien Kombination der Monosubstanzen, bei der in 15 bis 30 Minuten Abstand jeweils 2 Tropfen morgens und 2 Tropfen abends verabreicht werden müssen.

### Vorteile des Kombinationspräparates gegenüber der freien Kombination der Substanzen:

### 1. Verminderung der DPE-Konzentration

Bei dem erfindungsgemäßen Kombinationspräparat, das eine Betablockerkomponente enthält, ergibt sich durch die betablockerbedingte Verminderung der Kammervasserproduktion eine geänderte Pharmakokinetik und damit eine längere Bioverfügbarkeit der DPE-Komponente, die es möglicherweise erlaubt, die Konzentration der DPE-Komponente oder beider Komponenten bei gleicher Wirkungsdauer und Wirkungsstärke zu reduzieren bei 2 x täglicher Applikation des Kombinationspräparates. Das Kombinationspräparat ist prinzipiell mit einem Beta-1-selektiven (theoretisch besser geeignet), als auch mit allen nicht selektiven Beta-1-Beta-2-Blockern herstellbar. Die Wahl des Betablockers richtet sich danach, mit welchem der am Auge zugelassenen Betablocker, zusammen mit DPE hinsichtlich Stabilität und pH-Wert das beste Ergebnis bei der fixen Kombination erreicht wird (der pH-Wert ist für Penetration und lokale Verträglichkeit entscheidend). Beim Menschen läßt sich bei freier Kombination nicht selektiver Betablocker mit DPE eine gute additive, den Augeninnendruck senkende Wirkung erreichen, so daß die praktische Relevanz der Verwendung eines Beta-1-selektiven Betablockers bei einem DPE-Betablocker-Kombinationspräparat bisher am Menschen nicht abschließend beantwortet werden kann. Entscheidend ist, daß als Adrenalinkomponente ausschließlich das Prodrug des Adrenalins, das DPE verwendet wird, da es die geringeren lokalen und systemischen Nebenwirkungen im Vergleich zu anderen Adrenalinpräparaten bei gleicher Augeninnendurcksenkung aufweist.

Da im Präparat ein die Kammerwasserproduktion hemmendes und ein den Kammerwasserabfluß verbesserndes Medikament kombiniert werden, tritt eine Änderung der Pharmakokinetik ein, so daß die Konzentration von DPE von 0,1 % auf 0,09 % gesenkt werden kann, bei vergleichbarer drucksenkender Wirkung. Diese Erkenntnis ist neu und überraschend

### 2. Längere Wirkungsdauer und Aufhebung der Tachiphylaxie des Betablockers

Die fixe Kombination der Einzelkomponenten in Kombinationspräparaten hat eine längere Wirkungsdauer und höhere Drucksenkung zur Folge als die freie Kombination der Einzelsubstanzen. Der Wirkungsmechanismus der Kombinationspräparate scheint somit nicht nur die Summe der Einzelmechanismen der Monopräparate zu sein - ein überraschendes Phänomen, für das es bisher noch keine gesicherte pharmakologische Erklärung gibt. Die verlängerte und höhere drucksenkende Wirkung der Kombinationspräparate läßt sich nicht nur durch eine Reduzierung des gegenseitigen Auswascheffektes erklären, sondern läßt auch eine Änderung der Pharmakokinetik und/oder eine andere Interaktion der Medikamente im Auge vermuten. Bei Anwendung der Suspensionstechnologie läßt sich eine zusätzliche Reduzierung der Wirkstoffgesamtmenge erreichen, wodurch sich ein höheres Sicherheitsprofil hinsichtlich systemischer und lokaler Nebenwirkungen ergibt. Diese Suspensionstechnologie besteht aus Poly(Styrol-Divinylbenzol) Sulfonsäure (Amberlite IRP-69) 2,5 mg als Wirkstoffträger und Carbomer 934 P 2 mg zur Erhöhung der Depotfunktion. Inwieweit sich die Wirkstoffmenge unter Beibehaltung der 2 x täglichen Applikation bei gleicher Augeninnendrucksenkung für DPE und die nicht selektiven Betablocker reduzieren läßt bei Anwendung der Suspensionstechnologie, muß tierexperimentell geprüft werden, wobei die Reduzierung der Wirkstoffkonzentration bei der Betablockerkomponente von 0,5 % Betaxolol bei der Lösung auf 0,28 % Betaxolol bei der Suspension bei gleicher drucksenkender Wirkung gesichert ist.

### 3. Reduzierte Applikationsabhängigkeit (2 x täglich bzw. nach 10 Tagen 1 x täglich)

Ein entscheidender Vorteil des erfindungsgemäßen Kombinationspräparates ist für die wässerige Lösung wie auch für die Suspension die 2 x tägliche Anwendung aus nur einem Medikamentenfläschchen. Dies führt zu einem einfacheren Therapieschema und damit zu einer Verbesserung der Compliance bei höherer Wirkungsdauer und stärkerer Drucksenkung als bei der freien Kombination der Einzelsubstanzen.

Nach Erreichen einer ausreichenden Pigmentepithelbildung und Aufsättigung (nach ca. 10 Tagen) kann die Applikation sogar auf 1x morgens gesenkt werden. Wegen der im Schlaf deutlich reduzierten Kammerwasserproduktion muß die Gabe des Betablocker-DPE-Kombinationspräparates dann morgens erfolgen. In fixer Kombination läßt sich dann bei insgesamt reduzierter Wirkstoffmenge die Applikationshäufigkeit auf 1x morgens senken. Die sich daraus ergebenden Vorteile sind eine Verbesserung der Compliance, weitere Reduzierung der Nebenwirkungen, Erhöhung der Lebensqualität des Patienten usw. Die systemische Halbwertszeit in Stunden für Betaxolol (12 bis 20 Stunden) macht eine solche Reduzierung auf 1x täglich besonders wahrscheinlich, wobei die systemischen Halbwertszeiten auch für Carteolol (3 bis 7 Stunden), Levolbunolol (6 Stunden), Metipranolol (3 bis 4 Stunden), Timolol (3 bis 5 Stunden), auch eine solche 1x-Applikation erlauben werden, da die lokalen Halbwertszeiten am Auge bedeutend länger sind. In fixer Kombination steht somit bei reduzierter Medikamentengesamtdosis ein drucksenkendes Medikament mit 1x-Applikation zur Verfügung, da die DPE-Konzentration im Kammerwasser durch die zusätzliche Betablockerkomponente langsamer abfällt.

Betablocker (z.B. Timolol) führen zu einer Zunahme der Rezeptorendichte, was bei Daueranwendung langfristig zu einem Nachlassen der Wirkung des Betablockers führt. Adrenalin (DPE) führt dagegen zu einer Abnahme der Rezeptorendichte. Die Anwendung über lange Zeiträume ist somit bei fixer Kombination günstiger, da dann eine Tachiphylaxie vermutlich seltener ist. Das Erreichen einer Augeninnendrucksenkung über lange Zeiträume (Daueranwendung) ist bei fixer Kombination somit wahrscheinlicher.

### 4. Reduzierung der Nebenwirkungen

Durch die stets zeitgleiche Applikation fällt der Boluscharakter der konträren systemischen Nebenwirkungen weg und die kardiovaskulären und bronchopulmonalen Nebenwirkungen des Betablockers werden so durch die DPE-Komponente vermindert oder aufgehoben. Das Sicherheitsprofil liegt bei fixer Kombination somit höher.

### 5. Sofortige IOD-Senkung durch Aufhebung der DPE-Nachteile im Druckprofil

DPE führt während der 1. Stunde nach Applikation zu einer Zunahme der Kammerwasserbildung und damit zur Augeninnendruckerhöhung, während die Betablockerkomponente bereits in der 1. Stunde zur Abnahme der Kammerwasserbildung und damit zur Augeninnendrucksenkung führt. Dies ist von praktischer Bedeutung, da zwischen den Einzelapplikationen (bei freier Kombination) möglichst ein Abstand von ca. 30 Minuten liegen sollte, um die Gefahr des gegenseitigen Auswascheffektes unter anderem zu reduzieren. Die beiden in der 1. Stunde gegenläufigen Reaktionen heben sich in der fixen Kombination auf, so daß die fixe Kombination stets von Anfang an augeninnendrucksenkend wirkt. Dadurch wird eine gleichmäßigere Drucksenkung unter Reduzierung der Druckschwankungen erreicht. Der Unterschied des intraokularen Drucks zwischen maximalen und minimalen Druckwerten in der Tagesdruckkurve wird damit kleiner. Dies ist von Bedeutung, da bekannt ist, daß nicht nur die Höhe des Augeninnendruckes sondern auch die Höhe der Druckschwankung schädigend auf den Sehnerven wirkt. Diese Schwankungen werden bei fixer Kombination besser reduziert als mit freier Kombination. Erst nach der 1. Stunde wirken die beiden Substanzen gleichermaßen augeninnendrucksenkend. Nur bei fixer Kombination läßt sich durch die sicher stets zeitgleiche Applikation dieser Nachteil im Wirkprofil des DPE bzw. Adrenalins stets sicher ausgleichen.

### 6. Reduzierung der Konservierungsmittelbelastung

Bisher mußten bei freier Kombination pro Tag 2 Tropfen DPE (jeweils ein Tropfen morgens und ein Tropfen abends) und ebenso zwei Tropfen Betablocker (Summe: 4 Tropfen, die 4 "Einheiten" Konservierungsmittel enthalten) gegeben werden. Bei fixer Kombination sind nur noch 2 Tropfen und nach 10 Tagen meist nur noch 1 Tropfen morgens erforderlich, so daß nur noch zwei bzw. eine "Einheit" Konservierungsmittel das Auge erreicht. Die Gefahr der Epithelschädigung durch Konservierungsmittel ist durch die reduzierte Konservierungsmittelbelastung somit um 50 % bzw. 75 % geringer als bei freier Kombination und die lokale Verträglichkeit der fixen Kombination dadurch langfristig besser.

### 7. Ersatzmittel und neue Kombinationsmöglichkeiten

Die Pupillenweite liegt im Mittel bei der fixen Kombination von DPE und Betablocker bei 4,3 mm. Der Visus wird dadurch bei Linsentrübungen im Gegensatz zur Therapie mit Miotika nicht beeinträchtigt. (Pupillenweite im Vergleich zu den anderen fixen Kombinationen: Carbachol-Betablocker: 1,5 mm, DPE-Carbachol: 2 mm). Bei Glaukomen mit weitem Kammerwinkel stellt diese Kombination daher ein Ersatzmittel dar, wenn Miotika wegen Linsentrübung oder Allergie u.a. nicht einsetzbar sind.

Die fixe DPE-Betablockerkombination (2 Applikationen tägl. aus einer Flasche, nach ca. 10 Tagen sogar nur 1 x Applikation morgens) eröffnet neue krankheitsspezifische Drei- oder Vierkomponententherapien, so z.B. die freie Kombination mit Appraclonidin (1 x tägl.) oder lokalen Carboanhydrasehemmern, ebenfalls ohne Miosis. Eine Erweiterung des Spektrums der Medikamentenkombination wird damit erreicht, ohne daß insgesamt mehr als zwei Fläschen benötigt werden.

Die Einsatzbereiche des DPE-Betablocker-Kombinationspräparates sind z.B. schwer einstellbare Neovaskularisationsglaukome oder Sekun-därglaukome bei Iritis oder postoperative Augeninnendruckerhöhungen, bei denen Miotika - also z.B. Carbachol-Betablocker-Kombinationspräparate - kontraindiziert sind.

Das neue Kombinationspräparat kann die Stufenleiter einer compliancegerechten medikamentösen Kombinationstherapie verlängern und ist in Anbetracht der steigenden Lebenserwartung der Bevölkerung eine medizinische und ethische Notwendigkeit.

Die Konzentration an Dipivalylepinephrin in dem erfindungsgemäßen Präparat beträgt vorzugsweise 0,1 bis 0,09 % und die des Betablockers 0,5 bis 0,25 %.

Die Senkung der Konzentration des DPE's im Kombinationspräparat verbunden mit der weiteren Senkung auch der Betablockerkonzentration, insbesondere bei Anwendung der Suspensionstechnologie auch auf nicht selektive Betablocker, stellt ein neues, überraschendes Ergebnis dar und unterscheidet sich somit bei zumindest gleicher oder höherer Augeninnendrucksenkung und reduzierter Applikationshäufigkeit (1x morgens) grundsätzlich von der freien Kombination dieser Medikamente.

## Patentansprüche

1. Präparat zur Senkung des Augeninnendrucks auf der Basis von Dipivalylepinephrin, dadurch **gekennzeichnet,** daß es einen Beta-Blocker enthält.

2. Präparat nach Anspruch 1, dadurch **gekennzeichnet,** daß das Dipivalylepinephrin in einer Konzentration von 0,1 % bis 0,09 % vorliegt.

3. Präparat nach Anspruch 2, dadurch **gekennzeichnet,** daß der Betablocker als Beta-1-Beta-2-Blocker oder selektiver Beta-1-Blocker in einer Konzentration von 0,5 % bis 0,25 % vorliegt.

4. Kombinationspräparate nach Anspruch 1 bis 3, dadurch **gekennzeichnet,** daß sie in Form von Augentropfen (Lösung oder Suspension), Augensalbe, Augen-Gel, Augeninsert oder auf einem sonstigen Medikamententräger zur lokalen Applikation am Auge zubereitet sein können.
